# EUROPEAN PATENT APPLICATION

(11) **EP 4 834 365 A1**
(43) Date of publication of application: **04.11.2026**
(21) Application number: 24911472.9
(22) Date of filing: 27.12.2024
(51) Int. Cl.: A61K 39/395, A61K 38/18, A61P 27/02

(54) **PHARMACEUTICAL COMPOSITION CONTAINING ANTI-VEGF FUSION PROTEIN**

(30) Priority: 27.12.2023 CN 202311840919
(71) Applicant: Chengdu Kanghong Biotechnologies Co., Ltd., Chengdu, Sichuan 610036 (CN)
(72) Inventor: ZHOU, Mao, Chengdu, Sichuan 610036 (CN); LUO, Zuxiu, Chengdu, Sichuan 610036 (CN); DENG, Xing, Chengdu, Sichuan 610036 (CN); HUANG, Qian, Chengdu, Sichuan 610036 (CN); KE, Xiao, Chengdu, Sichuan 610036 (CN)
(74) Representative: Potter Clarkson
(86) International application number: PCT/CN2024/143188
(87) International publication number: WO 2025/140553

(57) **Abstract**

Provided is a pharmaceutical composition including an anti-VEGF fusion protein. The pharmaceutical composition includes an anti-VEGF fusion protein A and a protein fragment B. By controlling a content of the protein fragment B in the pharmaceutical composition to range from 0.1% to 5.4%, an aggregation rate of proteins in the composition can be reduced, stability of the proteins can be improved, and favorable biological activity can be maintained.

## Description

This application claims priority to Chinese Patent Application No. 2023118409197, filed on December 27, 2023, the entire contents of which are incorporated herein by reference.

### FIELD

The present disclosure relates to the field of pharmaceutical preparations, and in particular, to a pharmaceutical composition including an anti-VEGF fusion protein.

### BACKGROUND

Neovascularization is pivotal to the progression and spread of many diseases. A wide array of ocular diseases is associated with angiogenesis, including age-related macular degeneration (AMD), retinal vein occlusion (RVO), diabetic retinopathy (DR), and pathological myopia. Vascular endothelial growth factor (VEGF) is a highly specific growth factor that promotes vascular endothelial cell proliferation, which promotes increased vascular permeability, extracellular matrix denaturation, vascular endothelial cell migration, proliferation, and angiogenesis. VEGF is extensively distributed in multiple human and animal tissues. In healthy eyes, retinal pigment epithelial cells, vascular endothelial cells, and pericytes can synthesize lower levels of VEGF. A wealth of research has validated that excessive VEGF expression can trigger pathological neovascular ocular diseases. Given the importance of VEGF signal transduction in angiogenesis, curbing angiogenesis can be achieved by blocking VEGF or its receptor, which plays a significant therapeutic role in angiogenesis-related diseases including cancer, a retinal vascular disorder, and the like. Over the past decade and more, a number of anti-VEGF drugs for treating an ocular neovascular disease have been developed, such as bevacizumab, ranibizumab, aflibercept, conbercept, and other antibodies or fusion protein drugs.

Salt bridges, hydrogen bonds, disulfide bonds, and hydrophobic interactions are the forces that maintain the stability of protein conformation. The interactions between metal ions, substrates, cofactors, and other low molecular weight ligands also stabilize protein conformation. As is well known to those skilled in the art, antibodies or proteins are susceptible to various environmental factors during storage, such as temperature, humidity, oxygen, and ultraviolet radiation, which can cause various physical or chemical changes in fusion proteins, resulting in protein aggregation, fragmentation, oxidation, and denaturation. These changes can reduce the biological activity of the protein, diminish the therapeutic effect, and cause severe toxic and side effects.

### SUMMARY

One objective of the present disclosure is to provide a pharmaceutical composition including an anti-VEGF fusion protein with good stability and stable biological activity.

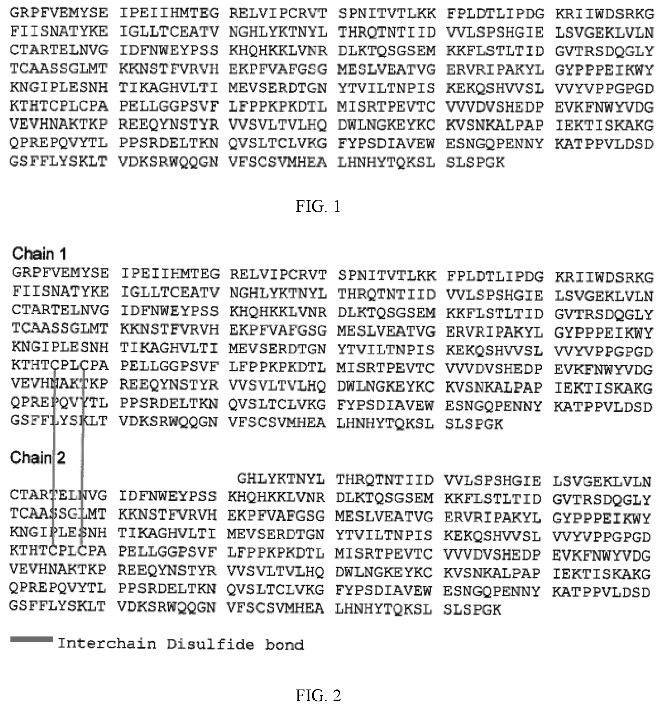

In some embodiments, the anti-VEGF fusion protein according to the present disclosure is a dimer including two fusion polypeptides, and each polypeptide includes an extracellular domain 2 of VEGFR-1, extracellular domains 3 and 4 of VEGFR-2, and a human immunoglobulin IgG4. In some specific embodiments, the anti-VEGF fusion protein according to the present disclosure is a dimer including two fusion polypeptides as set forth in SEQ ID NO: 1 (a sequence of SEQ ID NO: 1 is shown in FIG. 1). The two fusion polypeptides are linked via a disulfide bond, with a molecular weight of 142 kDa. This fusion protein is abbreviated below as a fusion protein A.

The fusion protein A is a fusion protein described in the Chinese patent Application No. ZL200610066257.2, entitled "APPLICATION OF VEGF RECEPTOR FUSION PROTEIN IN TREATING OCULAR DISEASE". Specifically, the fusion protein A is an active component of an FP3 fusion protein. It is constructed by fusing an immunoglobulin-like domain 2 of a human vascular endothelial growth factor (VEGF) receptor 1 and immunoglobulin-like domains 3 and 4 of a VEGF receptor 2 with a human immunoglobulin Fc fragment, and has an amino acid sequence as set forth in SEQ ID NO: 1. Therefore, the content of ZL200610066257.2 can be used for further description of the present disclosure.

It has been found in the present disclosure that other than the fusion protein A, proteins obtained through cellular expression or purification typically include protein fragments B, C, and D with low molecular weight. Such fragments are presumably induced under various stress conditions during cell culture and purification, which are commonly associated with covalent bond cleavage of proteins caused by a spontaneous or enzymatic reaction.

The protein fragment B is a dimer formed by one truncated fusion polypeptide and the other intact fusion polypeptide in the fusion protein A, with a molecular weight of 131.2 kDa. Specifically, the truncated fusion polypeptide in the protein fragment B has an amino acid sequence as set forth in positions 82 to 526 of SEQ ID NO: 1, and more specifically, has an amino acid sequence as set forth in SEQ ID NO: 2. The other intact fusion polypeptide in the protein fragment B has a full-length amino acid sequence as set forth in SEQ ID NO: 1. As shown in FIG. 2, the two fusion polypeptides of the protein fragment B are linked via a disulfide bond.

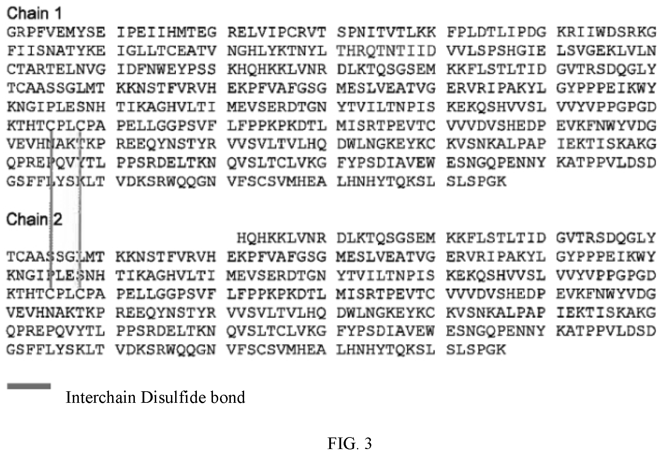

The protein fragment C is a dimer formed by one truncated fusion polypeptide and the other intact fusion polypeptide in the fusion protein A, with a molecular weight of 122.6 kDa. Specifically, the truncated fusion polypeptide in the protein fragment C has an amino acid sequence as set forth in positions 142 to 526 of SEQ ID NO: 1, and more specifically, has an amino acid sequence as set forth in SEQ ID NO: 3. The other intact fusion polypeptide in the protein fragment C has a full-length amino acid sequence as set forth in SEQ ID NO: 1. As shown in FIG. 3, the two fusion polypeptides of the protein fragment C are linked via a disulfide bond.

The protein fragment D is a fusion polypeptide (monomer) from the fusion protein A, and has a molecular weight of 71 kDa and an amino acid sequence as set forth in SEQ ID NO: 1.

The inventors of the present disclosure have surprisingly found that in the pharmaceutical compositions including the fusion protein A, a content of the protein fragment B has a significant impact on stability (e.g., an aggregation rate of the fusion protein A) and activity, whereas the protein fragments C and D have relatively minor impacts on the stability and activity. By controlling the content of the protein fragment B with low molecular weight in the pharmaceutical composition, stability and biological activity of the fusion protein A in the composition can be better maintained.

Therefore, on the one hand, the present disclosure provides a pharmaceutical composition. The pharmaceutical composition includes an anti-VEGF fusion protein A and 0.01% to 7% of a protein fragment B. In some preferred embodiments, the pharmaceutical composition includes the anti-VEGF fusion protein A and 0.01% to 5.4% of the protein fragment B. In some preferred embodiments, the pharmaceutical composition includes the anti-VEGF fusion protein A and 0.01% to 4.6% of the protein fragment B.

In some embodiments, a content of the protein fragment B according to the present disclosure ranges from 0.01% to 7%. In some preferred embodiments, the content of the protein fragment B according to the present disclosure ranges from 0.1% to 5.4%. In some preferred embodiments, the content of the protein fragment B according to the present disclosure ranges from 0.1% to 4.9%. In some preferred embodiments, the content of the protein fragment B according to the present disclosure ranges from 0.1% to 4.4%. In some preferred embodiments, the content of the protein fragment B according to the present disclosure ranges from 0.1% to 4.6%. In some preferred embodiments, the content of the protein fragment B according to the present disclosure ranges from 0.1% to 3.7%. In some preferred embodiments, the content of the protein fragment B according to the present disclosure ranges from 0.1% to 3.1%.

In some embodiments, the content of the protein fragment B according to the present disclosure ranges from 0.6% to 7%. In some preferred embodiments, the content of the protein fragment B according to the present disclosure ranges from 0.6% to 5.4%. In some preferred embodiments, the content of the protein fragment B according to the present disclosure ranges from 0.6% to 4.9%. In some preferred embodiments, the content of the protein fragment B according to the present disclosure ranges from 0.6% to 4.6%. In some preferred embodiments, the content of the protein fragment B according to the present disclosure ranges from 1.3% to 5.4%. In some preferred embodiments, the content of the protein fragment B according to the present disclosure ranges from 1.3% to 4.6%. In some preferred embodiments, the content of the protein fragment B according to the present disclosure ranges from 0.6% to 4.1%. In some preferred embodiments, the content of the protein fragment B according to the present disclosure ranges from 0.6% to 3.7%. In some preferred embodiments, the content of the protein fragment B according to the present disclosure ranges from 0.6% to 3.1%.

In some embodiments, in the present disclosure, the content of the protein fragment B ranges from 0.01% to 7%, a content of the protein fragment C ranges from 0% to 12.0%, and a content of the protein fragment D ranges from 0% to 6.1%. In some embodiments, in the present disclosure, the content of the protein fragment B ranges from 0.1% to 7%, the content of the protein fragment C ranges from 0% to 12.0%, and the content of the protein fragment D ranges from 0% to 6.1%. In some embodiments, in the present disclosure, the content of the protein fragment B ranges from 0.6% to 7%, the content of the protein fragment C ranges from 0% to 12.0%, and the content of the protein fragment D ranges from 0% to 6.1%. In some preferred embodiments, in the present disclosure, the content of the protein fragment B ranges from 0.6% to 5.4%, the content of the protein fragment C ranges from 0% to 12.0%, and the content of the protein fragment D ranges from 0% to 6.1%.

In some embodiments, purity of the anti-VEGF fusion protein A according to the present disclosure is greater than 77%. In some preferred embodiments, the purity of the anti-VEGF fusion protein A according to the present disclosure is greater than 78%, and preferably, greater than 78%, 79%, 80%, 81%, 82%, 83%, 84%, 85%, 86%, 87%, 88%, 89%, 90%, 91%, 92%, 93%, 94%, 95%, 96%, or 97%.

In some embodiments, the purity of the anti-VEGF fusion protein A according to the present disclosure ranges from 77% to 98%. In some preferred embodiments, the purity of the anti-VEGF fusion protein A according to the present disclosure ranges from 77% to 96%. In some preferred embodiments, the purity of the anti-VEGF fusion protein A according to the present disclosure ranges from 77% to 87%. In some embodiments, the purity of the anti-VEGF fusion protein A according to the present disclosure ranges from 80% to 98%. In some embodiments, the purity of the anti-VEGF fusion protein A according to the present disclosure ranges from 80% to 96%. In some preferred embodiments, the purity of the anti-VEGF fusion protein A according to the present disclosure ranges from 80% to 87%.

In some embodiments, the content of the fusion protein A and the contents of the protein fragments according to the present disclosure are determined by gel electrophoresis (SDS-PAGE). In some specific embodiments, the SDS-PAGE according to the present disclosure is performed in accordance with General Principle "0541 Electrophoresis Method, Fifth Method: SDS-Polyacrylamide Gel Electrophoresis" in Chinese Pharmacopoeia 2020 Edition (in 4 volumes*)*. For gel electrophoresis, a protein containing a sample loading buffer is subjected to water bath heating at 70±2°C for 10 minutes, and 4 µg of the treated sample is loaded onto a 4% to 15% precast polyacrylamide gel for electrophoresis and imaging. The precast gel is incorporated with a trichloride-based fluorescent dye, which covalently binds to tryptophan residues in the protein under ultraviolet irradiation of about 300 nm to emit fluorescence for visualizing the protein in the gel. The protein purity or impurity content is calculated based on response signal values of a principal component protein and an impure protein.

The fusion protein in the pharmaceutical composition of the present disclosure is prepared through the construction of an expression vector and the expression of cells (e.g., CHO cells) using conventional biological methods known in the art (e.g., a method described in CN200510073595.4). The contents of the fusion protein A and the protein fragments B, C, and D can be controlled through conventional purification methods in the art, such as a conventional purification method described in Protein Purification and Analysis Technology (compiled by Lu Jian, published by Beijing University of Technology Press) in 2005.

For example, in some embodiments, removal or content control of the protein fragments B, C, and D can be performed by gel filtration chromatography during purification of the fusion protein A. In the process of gel filtration chromatography, after a mixed protein sample containing components of different molecular weights is loaded onto a chromatographic column packed with gel particles, these components move along with the flow of an eluent. Within the column, two modes of movement occur: a vertical downward movement caused by gravity and random diffusion. Due to a larger diameter, a macromolecular protein cannot enter micropores within the gel particles, and can only flow through the gaps between the particles. Therefore, the macromolecular protein moves downward faster during elution, and is eluted from the gel column first. A small molecule protein can, in addition to diffusing in the gaps between the gel particles, penetrate into gel pores. During its downward movement, the small molecule protein continuously reciprocates between an interior of the gel and the gaps between the gel particles, resulting in a longer migration distance, and is ultimately eluted from the column. A medium-sized molecular protein elutes at a time intermediate between those of the macromolecular protein and the small molecule protein. The larger the molecule, the earlier it elutes, which ultimately causes the proteins of different molecular sizes to be separated from each other. In the present disclosure, a molecular weight of the fusion protein A is about 142 kDa, while molecular weights of the low-molecular-weight fragments B, C, and D range from 71 kDa to 131 kDa. A molecular weight of the fragment B is about 131.2 kDa, a molecular weight of the fragment C is about 122.6 kDa, and a molecular weight of the fragment D is about 71 kDa. Therefore, based on a difference between the molecular weights of the low-molecular-weight fragments and the molecular weight of the fusion protein A, gel chromatography can be used, and process parameters (e.g., packing material, column length, sample loading, flow rate, etc.) can be adjusted using the conventional method in the art, to separate a target protein from the low-molecular-weight fragments, thereby controlling contents of fragments with different molecular weights. The separation principle and process of the gel filtration chromatography are well known to those skilled in the art, and can be routinely adjusted based on an actual separation effect (see, e.g., Lu Jian, Protein Purification and Analysis Technology, Beijing: Chemical Industry Press, 2005, pp. 40-52). For example, in a specific exemplary embodiment, a chromatographic column is packed with a gel packing material suitable for separating proteins ranging from 10 kDa to 400 kDa, and is equilibrated with the equilibration buffer, to control a sample loading volume to be smaller than or equal to 10% CV and a sample loading flow rate to be smaller than or equal to 20 cm/h for sample loading, followed by equilibration and collection of the target protein. According to the above method, sample proteins with varying fragment contents can be obtained based on different peak collection parameters.

For example, in some embodiments, the contents of the protein A and the protein fragments B, C, and D can be controlled by cation exchange chromatography during the purification of the fusion protein A. Ion exchange chromatography is a commonly used purification method in protein purification technology. Its principle is that a charge carried by a separated substance can bind to an opposite charge carried by the packing material, and this binding interaction between the charged molecule and the packing material phase is reversible. When a pH value is changed or elution is performed using buffers of gradually increasing ionic strength, substances bound to the packing material can exchange ions with those in the eluent and be eluted into a solution. Since high-molecular-weight aggregates (HMWs), monomers, low-molecular-weight fragments (LMWs), host cell proteins (HCPs), and the like differ in charge, their binding capacities to the packing material are also different, resulting in different elution orders into the solution, and thus enabling their separation. In the present disclosure, since charge properties of the low-molecular-weight fragment and other impurities differ from a charge property of the protein A, contents of the low-molecular-weight fragments and other impurities in the fusion protein A are controlled. The separation principle and process of the cation exchange chromatography are well known to those skilled in the art, and can be routinely adjusted based on the actual separation effect (see, e.g., Lu Jian, Protein Purification and Analysis Technology, Beijing: Chemical Industry Press, 2005, pp. 64-119). For example, in a specific exemplary embodiment, the chromatographic column is packed with a strong cation exchange chromatography packing material, and the loaded sample is adjusted to weakly acidic conditions with conductivity lower than 30 mS/cm, followed by sample loading, equilibration, intermediate washing, and target protein elution. According to the above method, the sample proteins with varying fragment contents can be obtained based on the different peak collection parameters.

For example, in some embodiments, the contents of the protein A and the protein fragments B, C, and D can be controlled by mixed-mode chromatography during the purification of the fusion protein A. The mixed-mode chromatography involves performing an optimized design on a structure of a functional ligand, to combine two or more types of interaction modes. Generally, there are two main types of mixed-mode chromatography used for a protein-based biological product. One type is a cationic+hydrophobic mode, and the other type is an anionic+hydrophobic mode. The ligand binds to a target molecule through a variety of interactions, such as an ionic interaction, a hydrophobic interaction, and a hydrogen-bond interaction, and removes various impurities like aggregates, low-molecular-weight fragments, Protein A, HCPs, DNA, and viruses through a flow-through mode. The separation principle and process of the mixed-mode chromatography are well known to those skilled in the art, and can be routinely adjusted based on the actual separation effect. For example, in a specific exemplary embodiment, the chromatographic column is packed with a mixed-mode chromatography packing material possessing both anionic and hydrophobic properties, the loaded sample is adjusted to the weakly basic condition with conductivity ranging from 40 mS/cm to 60 mS/cm, and flow-through fraction is collected to obtain the target protein. According to the above method, the sample proteins with varying fragment contents can be obtained based on the different peak collection parameters.

In some embodiments, a concentration of the anti-VEGF fusion protein A according to the present disclosure ranges from 1 mg/mL to 200 mg/mL. In some preferred embodiments, the concentration of the anti-VEGF fusion protein A according to the present disclosure ranges from 10 mg/mL to 150 mg/mL, from 10 mg/mL to 140 mg/mL, from 10 mg/mL to 130 mg/mL, or from 10 mg/mL to 120 mg/mL. In some specific embodiments, the concentration of the anti-VEGF fusion protein A according to the present disclosure is 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, or 120 mg/mL.

In some embodiments, the pharmaceutical composition according to the present disclosure further includes a buffer, an osmotic pressure adjuster, amino acid, and/or a surfactant. A pH value of the pharmaceutical composition ranges from 6.8 to 8.7.

A suitable buffer for use in conjunction with the present disclosure includes, but is not limited to, an organic acid salt, such as Tris-HCl, citric acid, phosphate, histidine, succinate, or an acetate buffer.

A suitable osmotic pressure adjuster for use in conjunction with the present disclosure includes, but is not limited to, one or more of saccharides, glycerol, or propylene glycol. As saccharides, the osmotic pressure adjuster may include, but is not limited to, a monosaccharide, such as fructose, maltose, galactose, glucose, D-mannose, or sorbose; a disaccharide, such as lactose, sucrose, trehalose, or cellobiose; a polysaccharide, such as raffinose, melezitose, maltodextrin, glucan, or starch; and a polyol, such as mannitol, xylitol, maltitol, lactitol, xylitol, or sorbitol (glucitol). Preferably, the osmotic pressure adjuster is selected from one or more of sucrose, trehalose, mannitol, or sorbitol. More preferably, the osmotic pressure adjuster is selected from sucrose or trehalose. In some embodiments, the osmotic pressure adjuster of the present disclosure can also serve as a stabilizer, such as a saccharide.

A suitable surfactant for use in conjunction with the present disclosure includes, but is not limited to, a non-ionic surfactant, an ionic surfactant, and a zwitterionic surfactant. A typical surfactant used in the present disclosure includes, but is not limited to, a sorbitan fatty acid ester, a sorbitan trioleate, a glyceryl fatty acid ester, a polyglyceryl fatty acid ester, a polyoxyethylene sorbitan fatty acid ester, a polyoxyethylene glyceryl fatty acid ester, a polyethylene glycol fatty acid ester, a polyoxyethylene alkyl ether, a polyoxyethylene polyoxypropylene alkyl ether, a polyoxyethylene alkylphenyl ether, a polyoxyethylene hydrogenated castor oil (e.g., a polyoxyethylene castor oil or a polyoxyethylene hydrogenated castor oil), a polyoxyethylene beeswax derivative, a polyoxyethylene lanolin derivative, and a polyoxyethylene fatty acid amide; a C10-C18 alkyl sulfate (e.g., sodium cetyl sulfate, sodium dodecyl sulfate, or sodium oleyl sulfate), a polyoxyethylene sodium dodecyl sulfate, a sodium dodecyl sulfosuccinate, a propylene glycol, or a dimethyl sulfoxide; and a natural surfactant, such as a lecithin, a glycerophospholipid, or a sphingomyelin. A preferred surfactant is a polyoxyethylene sorbitan fatty acid ester, such as Polysorbate 20, 40, 60, or 80, or Poloxamer 188. A more preferred surfactant is Polysorbate 20 or 80.

Based on the stability of the fusion protein according to the present disclosure, the pH value of the pharmaceutical composition according to the present disclosure preferably ranges from 6.8 to 8.7. This pH range can be controlled by using the buffer or a pH adjuster. In some embodiments, the pH value of the pharmaceutical composition of the present disclosure ranges from 7.0 to 8.7. In some embodiments, the pH value of the pharmaceutical composition of the present disclosure ranges from 7.5 to 8.7. In some embodiments, the pH value of the pharmaceutical composition of the present disclosure ranges from 7.7 to 8.7. In an embodiment, the pH value of the pharmaceutical composition of the present disclosure is about 7.5, 7.6, 7.7, 7.8, 7.9, 8.0, 8.1, 8.2, 8.3, 8.4, 8.5, 8.6, or 8.7. In a preferred embodiment, the pH value of the pharmaceutical composition is about 7.7±0.2.

Suitable free amino acid used in the present disclosure includes, but is not limited to, arginine, lysine, histidine, ornithine, isoleucine, leucine, alanine, glycine, glutamic acid, or aspartic acid. Preferably, the free amino acid includes basic amino acid, i.e., arginine, lysine, and/or histidine. If the composition includes the histidine, the composition can act both as the buffer and as the free amino acid. However, when a histidine buffer is used, it is generally necessary to include non-histidine free amino acid, such as a histidine buffer and lysine/arginine. The amino acid may be present in any suitable salt form, such as hydrochloride, e.g., arginine-HCl. The pharmaceutical composition of the present disclosure may also include other expected excipients, such as an antioxidant, an antibacterial agent, etc.

On the other hand, the present disclosure provides a pharmaceutical composition. The pharmaceutical composition includes:1 mg/mL to 200 mg/mL of the fusion protein A; 5 mM to 300 mM of a buffer, in which the buffer is selected from one or more of Tris-HCl, citric acid, phosphate, histidine, glutamic acid, succinate, tromethamine, or an acetate buffer; 10 mM to 500 mM of amino acid, in which the amino acid is selected from one or more of lysine, arginine, histidine, ornithine, isoleucine, leucine, alanine, glycine, glutamic acid, or aspartic acid; 0% to 30% of an osmotic pressure adjuster, in which the osmotic pressure adjuster is selected from one or more of sucrose, trehalose, mannitol, glycerol, propylene glycol, or sorbitol; and 0% to 0.1% of a surfactant, in which the surfactant is selected from one or more of polyethylene glycol, Tween 20, Tween 80, Poloxamer 188 (P188), propylene glycol, or dimethyl sulfoxide. The pH value of the pharmaceutical composition ranges from 6.8 to 8.7.

In some more specific embodiments, the pharmaceutical composition includes: 1 mg/mL to 200 mg/mL of the fusion protein A; 5 mM to 300 mM of the buffer, in which the buffer is selected from one or more of citric acid, phosphate, arginine, glutamic acid, or tromethamine; 10 mM to 300 mM of the amino acid, in which the amino acid is selected from one or more of glutamic acid, arginine, or histidine; 0% to 30% of the osmotic pressure adjuster, in which the osmotic pressure adjuster is selected from one or more of sucrose, trehalose, mannitol, or sorbitol; and 0% to 0.1% of the surfactant, in which the surfactant is selected from one or more of Tween 20, Tween 80, or P188. The pH value of the pharmaceutical composition ranges from 6.8 to 8.7.

In some more specific embodiments, the pharmaceutical composition includes: 10 mg/mL to 150 mg/mL of the fusion protein A; 5 mM to 300 mM of the buffer, in which the buffer is selected from one or more of citric acid, phosphate, histidine, glutamic acid, or tromethamine; 100 mM to 300 mM of the amino acid, in which the amino acid is selected from one or more of glutamic acid, arginine, or histidine; 0% to 20% of the osmotic pressure adjuster, in which the osmotic pressure adjuster is selected from one or more of sucrose, trehalose, mannitol, or sorbitol; and 0% to 0.1% of the surfactant, in which the surfactant is selected from one or more of Tween 20, Tween 80, or P188. The pH value of the pharmaceutical composition ranges from 6.8 to 8.7.

In some more specific embodiments, the pharmaceutical composition includes: 10 mg/mL to 150 mg/mL of the fusion protein A; 10 mM to 250 mM of a citrate buffer; 100 mM to 250 mM of arginine or histidine; 5% to 20% of sucrose or trehalose; and 0% to 0.1% of Tween 20, Tween 80, or P188. The pH value of the pharmaceutical composition ranges from 7.5 to 8.7.

In some more specific embodiments, the pharmaceutical composition includes: 10 mg/mL to 120 mg/mL of the fusion protein A, 10 mM of a citrate buffer, 100 mM of arginine, 5% of sucrose, and 0.05% of Tween 20. The pH value of the pharmaceutical composition ranges from 7.5 to 8.7.

In some more specific embodiments, the pharmaceutical composition includes: 10 mg/mL to 120 mg/mL of the fusion protein A, 10 mM of a citrate buffer, 100 mM of arginine, and 5% of sucrose. The pH value of the pharmaceutical composition ranges from 7.5 to 8.7, and preferably, the pH value of the system is adjusted to 7.7 ± 0.2 using hydrochloric acid.

In some specific embodiments, the pharmaceutical composition includes: 10 mg/mL to 120 mg/mL of the fusion protein A, 100 mM of the citrate buffer, 250 mM of arginine, 20% of sucrose, and 0.1% of Tween 20. The pH value of the pharmaceutical composition ranges from 7.5 to 8.7.

In some specific embodiments, the pharmaceutical composition includes: 10 mg/mL to 120 mg/mL of the fusion protein A, 250 mM of the citrate buffer, 100 mM of histidine, 8% of sucrose, and 0.1% of Tween 20. The pH value of the pharmaceutical composition ranges from 7.5 to 8.7.

In some specific embodiments, the pharmaceutical composition includes: 10 mg/mL to 120 mg/mL of the fusion protein A, 10 mM of the citrate buffer, 250 mM of arginine, 20% of sucrose, and 0.1% of Tween 20. The pH value of the pharmaceutical composition ranges from 7.5 to 8.7.

In some specific embodiments, the pharmaceutical composition includes: 10 mg/mL to 120 mg/mL of the fusion protein A, 10 mM of tromethamine, 100 mM of arginine, and 5% of sucrose. The pH value of the pharmaceutical composition ranges from 7.5 to 8.7.

In some specific embodiments, the pharmaceutical composition includes: 10 mg/mL to 120 mg/mL of the fusion protein A, 290 mM of glutamic acid, 290 mM of arginine, and 78 mM of NaOH. The pH value of the pharmaceutical composition ranges from 7.5 to 8.7.

In some specific embodiments, the pharmaceutical composition includes: 10 mg/mL to 120 mg/mL of the fusion protein A, 5 mM of phosphate, and preferably, 5 mM of sodium dihydrogen phosphate, 100 mM of arginine, 10% of trehalose, and 0.01% of P188. The pH value of the pharmaceutical composition ranges from 7.5 to 8.7.

Another objective of the present disclosure is to provide a container or delivery device including the pharmaceutical composition according to the first objective of the present disclosure. In some specific embodiments, examples of the container includes, but is not limited to, a vial, a syringe, an ampoule, a bottle, a cartridge, and a sachet. The syringe may be administered through a standard barrel and needle, an automatic injector device, or a microinfusion device. In some preferred embodiments, the delivery device according to the present disclosure is a pre-filled injection device. A sufficient amount of the pharmaceutical composition formulated according to the present disclosure is held in a pre-filled container, which facilitates dispensing of a protein preparation for parenteral administration (injection or infusion). In some embodiments, the container or pre-filled container includes at least one pharmaceutical unit dosage form, which may be particularly suitable for self-administration. For example, a unit dose per vial, cartridge, or pre-filled container (e.g., a pre-filled syringe or disposable pen) may contain about 0.1 mL, 0.2 mL, 0.3 mL, 0.4 mL, 0.5 mL, 0.6 mL, 0.7 mL, 0.8 mL, 0.9 mL, 1 mL, 1.1 mL, 1.2 mL, 1.3 mL, 1.4 mL, 1.5 mL, 1.6 mL, 1.7 mL, 1.8 mL, 1.9 mL, 2.0 mL, 2.1 mL, 2.2 mL, 2.3 mL, 2.4 mL, 2.5 mL, 2.6 mL, 2.7 mL, 2.8 mL, 2.9 mL, 3.0 mL, 3.5 mL, 4.0 mL, 4.5 mL, 5.0 mL, 5.5 mL, 6.0 mL, 6.5 mL, 7.0 mL, 7.5 mL, 8.0 mL, 8.5 mL, 9.0 mL, 9.5 mL, or about 10.0 mL, or a greater volume of the pharmaceutical composition according to the present disclosure.

Another objective of the present disclosure is to provide a lyophilized preparation, prepared by lyophilizing the pharmaceutical composition according to the first objective of the present disclosure. The lyophilization method is well known to those skilled in the art, including, for example, sublimation of water from a frozen preparation under a controlled condition. The lyophilized preparation can be reconstituted into a solution, a suspension, an emulsion, or any other form suitable for administration or use. Reconstitution of the lyophilized preparation is typically achieved through the addition of an aqueous solution.

Another objective of the present disclosure is to provide use of the pharmaceutical composition according to the first objective of the present disclosure in the manufacture of a medicament for treating an ocular disease, or to provide the pharmaceutical composition according to the first objective of the present disclosure for treating an ocular disease. The "treatment" method includes administering the pharmaceutical composition of the present disclosure to a subject in need of such treatment (e.g., a subject suffering from an VEGF-mediated ocular disorder or a subject who may ultimately develop such a disorder), to prevent, cure, or delay the disorder or recurrence of the disorder, reduce severity of the disorder or the recurrent disorder, or ameliorate one or more symptoms of the disorder or the recurrent disorder.

In some specific embodiments, the ocular disease is an ocular neovascular disease. In some more specific embodiments, the ocular neovascular disease is selected from a retinal neovascular ocular disease, a choroidal neovascular ocular disease, an iris neovascular ocular disease, or a corneal neovascular ocular disease. In some preferred embodiments, the disease is selected from age-related macular degeneration, macular edema, macular edema secondary to retinal vein occlusion, retinal vein occlusion, macular edema caused by central retinal vein occlusion, macular edema caused by branch retinal vein occlusion, diabetic macular edema, diabetic retinopathy, polypoidal choroidal vasculopathy, choroidal neovascularization secondary to degenerative myopia, or retinopathy of prematurity. In other preferred embodiments, the disease is selected from age-related macular degeneration, diabetic macular edema, or diabetic retinopathy.

A therapeutic dosage can be easily determined by a doctor having ordinary skill in treating the disease or condition using known dosage adjustment techniques. For example, the therapeutically effective dose of the anti-VEGF fusion protein used in the pharmaceutical composition of the present disclosure is determined with consideration of a desired dose volume and an administration route. Typically, a therapeutically effective composition is administered at a concentration ranging from 10 mg/mL to about 200 mg/mL per dose. Preferably, the dosage employed in the method of the present disclosure ranges from about 100 mg/mL to about 120 mg/mL (i.e., about 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, or 120 mg/mL). In some preferred embodiments, the dosage of the anti-VEGF fusion protein employed in the method of the present disclosure is 10 mg/mL. In other preferred embodiments, the dosage of the anti-VEGF fusion protein employed in the method of the present disclosure is 120 mg/mL. Delivery of the pharmaceutical composition to the subject may be achieved via, for example, intravitreal injection, subretinal injection, choroidal injection (e.g., suprachoroidal injection), topical administration (e.g., eye drops), or by injection into recipient ocular tissue, to affect an eye of a mammal. In some embodiments, a dosage per eye ranges from at least about 0.5 mg up to about 10 mg. A preferred dosage per eye includes about 0.5 mg, 0.6 mg, 0.7 mg, 0.8 mg, 0.9 mg, 1.0 mg, 1.2 mg, 1.4 mg, 1.6 mg, 1.8 mg, 2.0 mg, 2.5 mg, 3.0 mg, 3.5 mg, 4.0 mg, 4.5 mg, 5.0 mg, 5.5 mg, 6.0 mg, 6.5 mg, 7 mg, 7.5 mg, 8 mg, 8.5 mg, 9 mg, 9.5 mg, or 10 mg. Such dosages can be delivered and administered in various volumes suitable for ocular administration.

Beneficial effects of the present disclosure are as follows:

A pharmaceutical composition including an anti-VEGF fusion protein A and a protein fragment B are provided. By controlling the content of the protein fragment B in the pharmaceutical composition, aggregation of the protein A in the pharmaceutical composition can be reduced, thereby improving stability. Moreover, the pharmaceutical composition can maintain good biological activity, and such biological activity can still be well maintained even after high-temperature treatment.

The polymer of the fusion protein A according to the present disclosure mainly refers to a polymer formed between the protein molecules via an intermolecular force, such as a covalent bond or a hydrogen bond. It is a complex composed of two or more proteins, such as a dimer, a tetramer, or a hexamer.

### BRIEF DESCRIPTION OF DRAWINGS

FIG. 1 shows a sequence (SEQ ID NO: 1) of a fusion protein A.
FIG. 2 is a schematic structural diagram of a protein fragment B.
FIG. 3 is a schematic structural diagram of a protein fragment C.

### DESCRIPTION OF EMBODIMENTS

An SEC-HPLC method according to the present disclosure is performed in accordance with General Principle "0514 Size Exclusion Chromatography" in Chinese Pharmacopoeia 2020 Edition (in 4 volumes*)*. A hydrophilic silica gel size exclusion chromatographic column TSK G3000 SWXL is used, with a sample loading quantity ranging from 50 µg to 200 µg. A mobile phase consists of 20 mM disodium hydrogen phosphate, 150 mM sodium chloride, and 200 mM arginine, with a pH value of 7.2 and a flow rate of 0.5 mL/min. The detection wavelength is set at 280 nm. A polymer content is calculated using an area normalization method. Unless otherwise specified, the polymer content (%) in the Examples of the present disclosure is determined by this SEC-HPLC method.

An SEC-UPLC method according to the present disclosure is performed in accordance with General Principle "0514 Size Exclusion Chromatography" in *Chinese Pharmacopoeia 2020 Edition (in 4 volumes)*. An ultra-high liquid phase and a size exclusion chromatographic column based on ethylidene bridge hybrid (BEH) particle technology (ACQUITY UPLC Protein BEH SEC Column, 200 A, 1.7 µm, 4.6 mm*150 mm) are used, with a sample loading quantity ranging from 4 µg to 12 µg. The mobile phase consists of 20 mM disodium hydrogen phosphate, 150 mM sodium chloride, and 200 mM arginine, with a pH value of 7.2, and a flow rate of 0.3 mL/min. The detection wavelength is set at 280 nm. The polymer content (%) is calculated using the area normalization method.

An SDS-PAGE staining-free method according to the present disclosure is performed in accordance with General Principle "0541 Electrophoresis Method, Fifth Method: SDS-Polyacrylamide Gel Electrophoresis" in Chinese Pharmacopoeia 2020 Edition (in 4 volumes*)*. For gel electrophoresis, a protein containing a sample loading buffer is subjected to water bath heating at 70±2°C for 10 minutes, and 4 µg of the treated sample is loaded onto a 4% to 15% precast polyacrylamide gel for electrophoresis and imaging. The precast gel is incorporated with a trichloride fluorescent dye, which covalently binds to tryptophan residues in the protein under ultraviolet irradiation of about 300 nm to emit fluorescence for visualizing the protein in the gel. The protein purity or impurity content is calculated based on response signal values of a principal component protein and an impure protein. Non-reducing protein purity (%) in the Examples of the present disclosure is determined by this SDS-PAGE method.

A luciferase reporter gene bioactivity assay according to the present disclosure is performed in accordance with General Principle "3535 Conbercept Bioactivity Assay" in Chinese Pharmacopoeia 2020 Edition (in 4 volumes*)*. This assay employs human embryonic kidney cells (HEK293) stably transfected with a vascular endothelial growth factor receptor 2 (VEGFR2) gene and a luciferase reporter gene luc2P. The biological activity of the protein is determined according to varying degrees of expression of cellular luciferase stimulated by vascular endothelial growth factor (VEGF) following blocking by proteins at varying concentrations. In the experiment, protein reference standard samples/test samples are serially diluted to 30,000 ng/mL, and then diluted to 1.21 ng/mL, resulting in a total of 11 concentration gradients. Each of the 11 graded reference standards/test samples is mixed with an equal volume of rhVEGF165 operating solution, and incubated at 37±1°C under 5% CO₂ for 20 minutes to 40 minutes, with duplicate wells made for each gradient. HEK293 cells are taken and prepared into a cell suspension at 5×10⁵ cells/mL using a DMEM test medium, and inoculated into a 96-well cell culture plate, with 80 µL per well. 20 µl of a mixed solution of different concentrations of the reference standards/test samples is added per well, followed by incubation at 37±1°C under 5% CO₂ for 5.8 hours to 6 hours. After equilibration at room temperature for 10 minutes to 15 minutes, 100 µL of a chromogenic substrate is dispensed into each well. After leaving at room temperature for 3 minutes to 5 minutes, the plate is immediately placed in a microplate reader, and a fluorescence response value of each well is measured using a chemiluminescence module. The rhVEGF165 operating solution is added to the cell hole as a positive control, and the DMEM test medium is added to the cell hole as a negative control. The same method is used to determine and record the experimental results. A computer program or a four-parameter regression calculation method is used for processing. A four-parameter curve is plotted with the concentrations of the test samples and reference standards on the abscissa and average luminescence response values on the ordinate, to calculate half effective concentrations (EC50) of the test samples and the reference standards. The relative biological potency of the test sample is calculated using the following formula: Relative biological potency (%) of test sample=EC50 of reference standard÷EC50 of test sample×100%. The biological activity (%) of the protein in the Examples of the present disclosure is evaluated by this method. The tested biological activity of all preparation samples prepared in the Examples of the present disclosure is higher than 85%.

All tested data in the Examples of the present disclosure are expressed as the average of six parallel samples (n=6).

### Example 1

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 7.7±0.2 using hydrochloric acid.

Proteins with different purities (referring to Table 1, the protein includes a fusion protein A and protein fragments B, C, and D, and specific contents of the components are shown in "Non-reducing Protein Purity (%)" in Table 1, the same below) were taken and prepared into samples 1 and 2 according to the above preparation composition. The samples were stored at 25°C, and polymer contents in the samples were determined by SEC-HPLC after 2 weeks and 4 weeks, respectively. The results were shown in Table 1. As can be seen from the results in Table 1, when the content of the protein fragment B was substantially consistent, the polymer contents of the two groups of samples remained substantially consistent after storage at 25°C for 2 weeks and 4 weeks, despite significant differences in contents of other components (e.g., the fusion protein A or protein fragments C and D). Meanwhile, biological activity of the proteins after storage at 35°C for 15 days was determined. The results showed that the biological activity of the proteins in both groups of samples was greater than 80%, indicating good maintenance of activity.

**[Table 1]**

| Sample | Non-reducing Protein Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 1 | 86.7 | 2.3 | 8.1 | 2.1 | 0.8 | 1.8 | 2.7 |
| 2 | 93.0 | 2.5 | 2.6 | 1.7 | 0.2 | 1.9 | 2.7 |

### Example 2

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 7.7±0.2 using hydrochloric acid.

Proteins with different purities (see Table 2) were taken and prepared into samples 3 and 4 according to the above preparation composition. The samples were stored at 25°C, and polymer contents in the samples were determined by SEC-HPLC after 2 weeks and 4 weeks, respectively. The results were shown in Table 2. As can be seen in Table 2, with the increase in the content of the protein fragment B in the preparation samples, the polymer contents in the samples increased more significantly after storage, and aggregation rates of the polymers were faster. Meanwhile, the biological activity of the proteins after storage at 35°C for 15 days was determined. The biological activity of the protein in the sample 3 was greater than 75%, and the biological activity of the protein in the sample 4 was lower than 65%.

**[Table 2]**

| Sample | Non-reducing Protein Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 3 | 82.3 | 6.1 | 7.0 | 3.9 | 0.7 | 3.4 | 4.8 |
| 4 | 79.4 | 9.3 | 7.2 | 4.0 | 0.1 | 4.5 | 6.3 |

### Example 3

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 7.7±0.2 using hydrochloric acid.

Proteins with different purities (see Table 3) were taken and prepared into samples 5 and 6 according to the above preparation composition. The samples were stored at 25°C, and polymer contents in the samples were determined by SEC-HPLC after 2 weeks and 4 weeks, respectively. The results were shown in Table 3. As can be seen from the results in Table 3, when the content of the protein fragment B is substantially consistent, polymer contents of the two groups of samples remained substantially consistent after storage at 25°C, despite significant differences in contents of other components (e.g., a protein fragment C). Meanwhile, the biological activity of the proteins of the two groups of samples after storage at 35°C for 15 days was determined. The results showed that the biological activity of the proteins in both groups of samples was greater than 80%.

**[Table 3]**

| Sample | Non-reducing Protein Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 5 | 82.8 | 3.8 | 6.5 | 6.1 | 0.8 | 2.7 | 4.1 |
| 6 | 80.0 | 4.0 | 9.1 | 6.0 | 0.9 | 2.8 | 4.1 |

### Example 4

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 7.7±0.2 using hydrochloric acid.

Proteins with different purities (see Table 4) were taken and prepared into samples 7 and 8 according to the above preparation composition. The samples were stored at 25°C, and polymer contents in the samples were determined by SEC-HPLC after 2 weeks and 4 weeks, respectively. The results were shown in Table 4. As can be seen in Table 4, when the content of the protein fragment B in the composition was substantially consistent, polymer contents of the two groups of samples remained substantially consistent, despite significant differences in contents of other components (e.g., proteins A, C, and D). Meanwhile, the biological activity of the proteins of the two groups of samples after storage at 35°C for 15 days was determined. The results showed that the biological activity of the proteins in both groups of samples was greater than 80%.

**[Table 4]**

| Sample | Non-reducing Protein Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 7 | 87.0 | 4.4 | 5.0 | 2.9 | 0.7 | 2.9 | 4.2 |
| 8 | 77.1 | 4.6 | 11.4 | 6.0 | 0.9 | 2.9 | 4.3 |

### Example 5

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 7.7±0.2 using hydrochloric acid.

Proteins with different purities (see Table 5) were taken and prepared into samples 9 to 15 according to the above preparation composition. The samples were stored at 25°C, and polymer contents in the samples were determined by SEC-HPLC after 2 weeks and 4 weeks, respectively. The results were shown in Table 5. As can be seen in Table 5, with the increase in the content of the protein fragment B in the composition, polymer contents in the preparation samples increased more significantly after storage, and the aggregation rate of the polymer was faster. Meanwhile, the biological activity of the proteins of the samples after storage at 35°C for 15 days was determined. The biological activity of the proteins in the samples 9 to 13 was greater than 80%, the biological activity of the protein in the sample 14 was greater than 75%, and the biological activity of the protein in the sample 15 was lower than 65%.

**[Table 5]**

| Sample | Non-reducing Protein Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 9 | 97.6 | 0.6 | 0.6 | 1.2 | 0 | 1.3 | 2.0 |
| 10 | 97.3 | 0.8 | 0.0 | 1.3 | 0.6 | 1.3 | 2.1 |
| 11 | 91.4 | 1.5 | 4.7 | 1.8 | 0.6 | 1.7 | 2.4 |
| 12 | 81.5 | 3.1 | 12.0 | 2.6 | 0.8 | 1.9 | 2.8 |
| 13 | 81.5 | 5.4 | 6.5 | 5.2 | 1.4 | 3.1 | 4.5 |
| 14 | 78.2 | 6.9 | 8.2 | 5.6 | 1.1 | 4.0 | 5.4 |
| 15 | 78.2 | 8.0 | 8.4 | 4.7 | 0.7 | 4.1 | 5.8 |

### Example 6

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 7.7±0.2 using hydrochloric acid.

Proteins with different purities (see Table 6) were taken and prepared into samples 16 to 23 according to the above preparation composition. Meanwhile, the biological activity of the proteins of the samples after storage at 35°C for 15 days was determined, and details thereof were shown in Table 6.

**[Table 6]**

| Sample | Non-reducing Protein Purity (%) | | | | Biological Activity (%) |
|---|---|---|---|---|---|
| | Fusion Protein A | Protein Fragment B | Protein Fragment C | Protein Fragment D | |
| 16 | 95.2 | 1.3 | 1.6 | 1.9 | 88 |
| 17 | 92.2 | 2.8 | 2.6 | 2.4 | 89 |
| 18 | 88.2 | 3.7 | 5.5 | 2.6 | 84 |
| 19 | 85.0 | 4.1 | 7.4 | 3.5 | 85 |
| 20 | 88.0 | 4.9 | 3.8 | 3.3 | 82 |
| 21 | 80.4 | 5.4 | 10.1 | 4.1 | 80 |
| 22 | 80.7 | 8.4 | 7.5 | 3.4 | 60 |
| 23 | 72.8 | 12.6 | 9.8 | 4.8 | 58 |

### Example 7

Composition of the preparation sample was as follows:

| | |
|---|---|
| Protein | 10 mg/mL |
| Citric acid | 10 mM |
| Arginine | 100 mM |
| Trehalose | 5%. |

A pH value of the system was adjusted to 7.7±0.2 using hydrochloric acid.

Proteins with different purities (see Table 7) were taken and prepared into samples 24 and 25 according to the above preparation composition. The samples were stored at 25°C, and polymer contents in the samples were determined by SEC-HPLC after 2 weeks and 4 weeks, respectively. The results were shown in Table 7. Meanwhile, the biological activity of the proteins of the samples after storage at 35°C for 15 days was determined. The results showed that the biological activity of the proteins in both groups of samples was greater than 80%.

**[Table 7]**

| Sample | Non-reducing Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 24 | 96.0 | 1.0 | 1.1 | 1.8 | 0.1 | 1.3 | 2.0 |
| 25 | 89.3 | 4.0 | 3.8 | 2.9 | 0 | 1.9 | 2.9 |

### Example 8

Composition of the preparation sample was as follows:

| | |
|---|---|
| Protein | 10 mg/mL |
| Tromethamine | 10 mM |
| Arginine | 100 mM |
| Sucrose | 5%. |

A pH value of the system was adjusted to 7.7±0.2.

Proteins with different purities (see Table 8) were taken and prepared into samples 26 and 27 according to the above preparation composition. The samples were stored at 25°C, and polymer contents in the samples were determined by SEC-HPLC after 2 weeks and 4 weeks, respectively. The results were shown in Table 8. Meanwhile, the biological activity of the proteins of the samples after storage at 35°C for 15 days was determined. The results showed that the biological activity of the proteins in both groups of samples was greater than 80%.

**[Table 8]**

| Sample | Non-reducing Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 26 | 95.9 | 0.7 | 1.4 | 2.0 | 0 | 1.3 | 2.0 |
| 27 | 89.4 | 4.2 | 3.5 | 2.8 | 0.1 | 1.6 | 2.4 |

### Example 9

Composition of the preparation sample was as follows:

| | |
|---|---|
| Protein | 10 mg/mL |
| Glutamic acid | 290 mM |
| Arginine | 290 mM |
| NaOH | 78mM |

A pH value of the system was adjusted to 7.7±0.2.

Proteins with different purities (see Table 9) were taken and prepared into samples 28 and 29 according to the above preparation composition. The samples were stored at 25°C, and polymer contents in the samples were determined by SEC-HPLC after 2 weeks and 4 weeks, respectively. The results were shown in Table 9. Meanwhile, the biological activity of the proteins of the samples after storage at 35°C for 15 days was determined. The results showed that the biological activity of the proteins in both groups of samples was greater than 80%.

**[Table 9]**

| Sample | Non-reducing Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 28 | 96.1 | 0.7 | 1.1 | 2.1 | 0 | 0.8 | 1.1 |
| 29 | 88.9 | 4.1 | 3.9 | 3.0 | 0.1 | 1.3 | 1.8 |

### Example 10

Composition of the preparation sample was as follows:

| | |
|---|---|
| Protein | 10 mg/mL |
| Sodium dihydrogen phosphate | 5 mM |
| Arginine | 100 mM |
| Trehalose | 10% |
| P188 | 0.01%. |

A pH value of the system was adjusted to 7.7±0.2 using hydrochloric acid.

Proteins with different purities (see Table 10) were taken and prepared into samples 30 and 31 according to the above preparation composition. The samples were stored at 25°C, and polymer contents in the samples were determined by SEC-HPLC after 2 weeks and 4 weeks, respectively. The results were shown in Table 10. Meanwhile, the biological activity of the proteins of the samples after storage at 35°C for 15 days was determined. The results showed that the biological activity of the proteins in both groups of samples was greater than 80%.

**[Table 10]**

| Sample | Non-reducing Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 30 | 96.3 | 0.8 | 1.1 | 1.7 | 0.1 | 0.8 | 1.1 |
| 31 | 89.1 | 4.2 | 3.9 | 2.8 | 0 | 1.4 | 1.9 |

### Example 11

Composition of the preparation sample was as follows:

| | |
|---|---|
| Protein | 10 mg/mL |
| Citric acid | 100 mM |
| Arginine | 250 mM |
| Sucrose | 20% |
| Tween 20 | 0.1%. |

A pH value of the system was adjusted to 7.7±0.2.

Proteins with different purities (see Table 11) were taken and prepared into samples 32 and 33 according to the above preparation composition. The samples were stored at 25°C, and polymer contents in the samples were determined by SEC-HPLC after 2 weeks and 4 weeks, respectively. The results were shown in Table 11. Meanwhile, the biological activity of the proteins after storage at 35°C for 15 days was determined. The results showed that the biological activity of the proteins in both groups of samples was greater than 80%.

**[Table 11]**

| | Non-reducing Purity (%) | | | | | Polymer Content (%) | |
|---|---|---|---|---|---|---|---|
| Sample | Fusion Protein A | Protein Fragment B | Protein Fragment C | Protein Fragment D | Others | 2 W | 4 W |
| 32 | 96.1 | 0.8 | 1.1 | 2.0 | 0 | 0.5 | 0.6 |
| 33 | 89.0 | 4.2 | 3.8 | 3.0 | 0 | 0.8 | 0.9 |

### Example 12

Composition of the preparation sample was as follows:

| | |
|---|---|
| Protein | 10 mg/mL |
| Citric acid | 250 mM |
| Histidine | 100 mM |
| Sucrose | 8% |
| Tween 20 | 0.1%. |

A pH value of the system was adjusted to 7.7±0.2.

Proteins with different purities (see Table 12) were taken and prepared into samples 34 and 35 according to the above preparation composition. The samples were stored at 25°C, and polymer contents in the samples were determined by SEC-HPLC after 2 weeks and 4 weeks, respectively. The results were shown in Table 12. Meanwhile, the biological activity of the proteins in the samples after storage at 35°C for 15 days was determined. The results showed that the biological activity of the proteins in both groups of samples was greater than 80%.

**[Table 12]**

| Sample | Non-reducing Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 34 | 96.0 | 0.8 | 1.2 | 2.0 | 0 | 0.7 | 0.8 |
| 35 | 88.9 | 4.4 | 3.9 | 2.8 | 0 | 1.2 | 1.5 |

### Example 13

Composition of the preparation sample was as follows:

| | |
|---|---|
| Protein | 20 mg/mL |
| Citric acid | 10 mM |
| Arginine | 250 mM |
| Sucrose | 20% |
| Tween 20 | 0.1%. |

A pH value of the system was adjusted to 7.9±0.2 using hydrochloric acid.

Proteins with different purities (see Table 13) were taken and prepared into samples 36 and 37 according to the above preparation composition. The samples were stored at 25°C, and polymer contents in the samples were determined by SEC-HPLC after 2 weeks and 4 weeks, respectively. The results were shown in Table 13. Meanwhile, the biological activity of the proteins in the samples after storage at 35°C for 15 days was determined. The results showed that the biological activity of the proteins in both groups of samples was greater than 80%.

**[Table 13]**

| Sample | Non-reducing Purity (%) | | | | | Polymer Content (%) | |
|---|---|---|---|---|---|---|---|
| | Fusion Protein A | Protein Fragment B | Protein Fragment C | Protein Fragment D | Others | 2 W | 4 W |
| 36 | 95.8 | 0.9 | 1.1 | 2.1 | 0.1 | 0.9 | 1.3 |
| 37 | 89.0 | 4.1 | 3.9 | 2.9 | 0.1 | 1.6 | 2.1 |

### Example 14

Composition of the preparation sample was as follows:

| | |
|---|---|
| Protein | 120 mg/mL |
| Citric acid | 10 mM |
| Arginine | 100 mM |
| Sucrose | 5% |
| Tween 20 | 0.1%. |

A pH value of the system was adjusted to range from 7.7 to 8.7 using hydrochloric acid.

Proteins with different purities (see Table 14) were taken and prepared into samples 38 to 40 according to the above preparation composition. The samples were stored at 25°C, and polymer contents in the samples were determined by SEC-HPLC after 2 weeks and 4 weeks, respectively. The results were shown in Table 14.

**[Table 14]**

| Sample | Non-reducing Purity (%) | | | | | Polymer Content (%) |
|---|---|---|---|---|---|---|
| | Fusion Protein A | Protein Fragment B | Protein Fragment C | Protein Fragment D | Others | 2 W |
| 38 | 97.5 | 0.8 | 0.7 | 1.0 | 0.0 | 12.6 |
| 39 | 93.5 | 2.3 | 2.4 | 1.8 | 0.0 | 13.7 |
| 40 | 77.6 | 9.5 | 8.2 | 4.6 | 0.1 | 20.2 |

### Example 15

Composition of the preparation sample was as follows:

| | |
|---|---|
| Protein | 10 mg/mL |
| Citric acid | 10 mM |
| Arginine | 100 mM |
| Sucrose | 5% |
| Tween 20 | 0.05%. |

A pH value of the system was adjusted to 5±0.5 using hydrochloric acid.

Proteins with different purities in Table 15 were taken and prepared into samples 41 to 44 according to the above preparation composition. Then, polymer contents in the samples were determined by SEC-UPLC. The results were shown in Table 15.

**[Table 15]**

| | Non-reducing Purity (%) | | | | | Polymer Content (%) |
|---|---|---|---|---|---|---|
| Sample | Fusion Protein A | Protein Fragment B | Protein Fragment C | Protein Fragment D | Others | |
| 41 | 93.5 | 1.7 | 3.2 | 1.6 | 0.0 | 0.6 |
| 42 | 85.5 | 5.3 | 4.7 | 4.5 | 0.0 | 2.9 |
| 43 | 78.2 | 8.1 | 10.2 | 3.5 | 0.0 | 4.8 |
| 44 | 71.4 | 11.5 | 8.9 | 8.2 | 0.0 | 6.7 |

### Example 16: Protein Purification

### 1. Gel Chromatography

A chromatographic column was packed with a gel packing material suitable for separating proteins ranging from 10 kDa to 400 kDa, and was equilibrated with a chromatographic equilibration buffer, to control a sample loading volume to be smaller than or equal to 10% CV and a sample loading flow rate to be smaller than or equal to 20 cm/h for sample loading, followed by equilibration and collection of a target protein. Proteins in samples 1 to 4, 9 to 23, and 41 to 44 were obtained based on different peak collection parameters.

### 2. Cation Exchange Chromatography

The chromatographic column was packed with a strong cation exchange chromatography packing material, and the loaded sample was adjusted to a weakly acidic condition with conductivity lower than 30 mS/cm, followed by sample loading, equilibration, intermediate washing, and target protein elution. Proteins of samples 5, 6, and 38 to 40 were obtained based on the different peak collection parameters.

### 3. Mixed-Mode Chromatography

The chromatographic column was packed with a mixed-mode chromatography packing material possessing both anionic and hydrophobic properties, the loaded sample was adjusted to the weakly acidic condition with conductivity ranging from 40 mS/cm to 60 mS/cm, and flow-through fraction was collected to obtain the target protein. Proteins of samples 7, 8, and 24 to 37 were obtained based on the different peak collection parameters.

## Claims

1. A pharmaceutical composition, comprising:
an anti-VEGF fusion protein A, wherein the fusion protein A is a dimer comprising two fusion polypeptides as set forth in SEQ ID NO: 1; and
a protein fragment B, wherein the protein fragment B is a dimer comprising two fusion polypeptides as set forth in SEQ ID NO: 1 and SEQ ID NO: 2, respectively, and wherein a content of the protein fragment B in the pharmaceutical composition ranges from 0.01% to 7%, and preferably, from 0.01% to 5.4%, and more preferably, from 0.01% to 4.6%.

2. The pharmaceutical composition according to claim 1, wherein the content of the protein fragment B in the pharmaceutical composition ranges from 0.6% to 7%, preferably, from 0.6% to 5.4%, and more preferably, from 0.6% to 4.6% or from 1.3% to 5.4%; and even more preferably, from 1.3% to 4.6%.

3. The pharmaceutical composition according to claim 1, wherein a content of the fusion protein A in the pharmaceutical composition ranges from 77% to 98%, and preferably, from 80% to 98%.

4. The pharmaceutical composition according to claim 3, wherein the content of the fusion protein A in the pharmaceutical composition ranges from 77% to 87%, and preferably, from 80% to 87%.

5. The pharmaceutical composition according to claim 1, wherein a concentration of the fusion protein A in the pharmaceutical composition ranges from 1 mg/mL to 200 mg/mL, preferably, from 10 mg/mL to 150 mg/mL, and more preferably, from 10 mg/mL to 120 mg/mL.

6. The pharmaceutical composition according to claim 1, wherein a pH value of the pharmaceutical composition ranges from 6.8 to 8.7, preferably, from 7.5 to 8.7, and more preferably 7.7±0.2.

7. The pharmaceutical composition according to any one of claims 1 to 6, further comprising:
a buffer;
an osmotic pressure adjuster;
a surfactant; and
amino acid,
wherein a pH value of the pharmaceutical composition ranges from 6.8 to 8.7.

8. The pharmaceutical composition according to claim 6, comprising:
1 mg/mL to 200 mg/mL of the fusion protein A;
5 mM to 300 mM of a buffer, wherein the buffer is selected from one or more of Tris-HCl, citric acid, phosphate, histidine, glutamic acid, succinate, tromethamine, or an acetate buffer;
10 mM to 500 mM of amino acid, wherein the amino acid is selected from one or more of lysine, arginine, histidine, ornithine, isoleucine, leucine, alanine, glycine, glutamic acid, or aspartic acid;
0% to 30% of an osmotic pressure adjuster, wherein the osmotic pressure adjuster is selected from one or more of sucrose, trehalose, mannitol, glycerol, propylene glycol, or sorbitol; and
0% to 0.1% of a surfactant, wherein the surfactant is selected from one or more of polyethylene glycol, Tween 20, Tween 80, P188, propylene glycol, or dimethyl sulfoxide,
wherein the pH value of the pharmaceutical composition ranges from 6.8 to 8.7.

9. The pharmaceutical composition according to claim 7, comprising:
1 mg/mL to 200 mg/mL of the fusion protein A;
5 mM to 300 mM of the buffer, wherein the buffer is selected from one or more of citric acid, phosphate, arginine, glutamic acid, or tromethamine;
10 mM to 300 mM of the amino acid, wherein the amino acid is selected from one or more of glutamic acid, arginine, or histidine;
0% to 30% of the osmotic pressure adjuster, wherein the osmotic pressure adjuster is selected from one or more of sucrose, trehalose, mannitol, or sorbitol; and
0% to 0.1% of the surfactant, wherein the surfactant is selected from one or more of Tween 20, Tween 80, or P188,
wherein the pH value of the pharmaceutical composition ranges from 6.8 to 8.7.

10. The pharmaceutical composition according to claim 8, comprising:
10 mg/mL to 150 mg/mL of the fusion protein A;
10 mM to 250 mM of a citrate buffer;
100 mM to 250 mM of arginine or histidine;
5% to 20% of sucrose or trehalose; and
0% to 0.1% of Tween 20, Tween 80, or P188,
wherein the pH value of the pharmaceutical composition ranges from 7.5 to 8.7.

11. The pharmaceutical composition according to claim 1, comprising:
10 mg/mL to 120 mg/mL of the fusion protein A, 10 mM of a citrate buffer, 100 mM of arginine, 5% of sucrose, and 0.05% of Tween 20, wherein a pH value of the pharmaceutical composition ranges from 7.5 to 8.7; or
10 mg/mL to 120 mg/mL of the fusion protein A, 100 mM of the citrate buffer, 250 mM of arginine, 20% of sucrose, and 0.1% of Tween 20, wherein the pH value of the pharmaceutical composition ranges from 7.5 to 8.7; or
10 mg/mL to 120 mg/mL of the fusion protein A, 250 mM of the citrate buffer, 100 mM of histidine, 8% of sucrose, and 0.1% of Tween 20, wherein the pH value of the pharmaceutical composition ranges from 7.5 to 8.7; or
10 mg/mL to 120 mg/mL of the fusion protein A, 10 mM of the citrate buffer, 250 mM of arginine, 20% of sucrose, and 0.1% of Tween 20, wherein the pH value of the pharmaceutical composition ranges from 7.5 to 8.7; or
10 mg/mL to 120 mg/mL of the fusion protein A, 10 mM of tromethamine, 100 mM of arginine, and 5% of sucrose, wherein the pH value of the pharmaceutical composition ranges from 7.5 to 8.7; or
10 mg/mL to 120 mg/mL of the fusion protein A, 290 mM of glutamic acid, 290 mM of arginine, and 78 mM of NaOH, wherein the pH value of the pharmaceutical composition ranges from 7.5 to 8.7; or
10 mg/mL to 120 mg/mL of the fusion protein A, 5 mM of phosphate, and preferably, 5 mM of sodium dihydrogen phosphate, 100 mM of arginine, 10% of trehalose, and 0.01% of P188, wherein the pH value of the pharmaceutical composition ranges from 7.5 to 8.7; or
10 mg/mL to 120 mg/mL of the fusion protein A, 10 mM of the citrate buffer, 100 mM of arginine, and 5% of trehalose, wherein the pH value of the pharmaceutical composition ranges from 7.5 to 8.7.

12. The pharmaceutical composition according to claim 1, wherein a content of the fusion protein A or the content of the protein fragment B is determined by gel electrophoresis (SDS-PAGE).

13. A container or delivery device, comprising the pharmaceutical composition according to any one of claims 1 to 12.

14. The container or delivery device according to claim 13, wherein the container is a vial or a syringe.

15. The container or delivery device according to claim 13, wherein the delivery device is a pre-filled injection device.

16. A lyophilized preparation, prepared by lyophilizing the pharmaceutical composition according to any one of claims 1 to 12.

17. Use of the pharmaceutical composition according to any one of claims 1 to 12 in the manufacture of a medicament for treating an ocular disease.

18. A method for treating an ocular disease, comprising:
administering the pharmaceutical composition according to any one of claims 1 to 12 to a subject.

19. The use according to claim 17 or the method according to claim 18, wherein the ocular disease is an ocular neovascular disease, and preferably, the ocular neovascular disease is selected from a retinal neovascular ocular disease, a choroidal neovascular ocular disease, an iris neovascular ocular disease, or a corneal neovascular ocular disease.

20. The use or method according to claim 19, wherein:
the ocular neovascular disease is selected from age-related macular degeneration, macular edema, macular edema secondary to retinal vein occlusion, retinal vein occlusion, macular edema caused by central retinal vein occlusion, macular edema caused by branch retinal vein occlusion, diabetic macular edema, diabetic retinopathy, polypoidal choroidal vasculopathy, choroidal neovascularization secondary to degenerative myopia, or retinopathy of prematurity; and
preferably, the ocular neovascular disease is selected from age-related macular degeneration, diabetic macular edema, or diabetic retinopathy.
